# EUROPÄISCHE PATENTSCHRIFT

(11) **EP 1 789 445 B1**
(45) Veröffentlichungstag und Bekanntmachung des Hinweises auf die Patenterteilung: **17.09.2008**
(21) Anmeldenummer: 05786772.3
(22) Anmeldetag: 09.09.2005
(51) Int. Cl.: C07K 14/78, C07K 14/745, C07K 1/30

(54) **VERFAHREN ZUR ENTFERNUNG VON FIBRONEKTIN AUS PLASMAFRAKTIONEN**
METHOD FOR REMOVING FIBRONECTIN FROM PLASMA FRACTIONS
PROCEDE D'EXTRACTION DE FIBRONECTINE DANS DES FRACTIONS DE PLASMA

(30) Priorität: 14.09.2004 DE 102004044429
(43) Veröffentlichungstag der Anmeldung: 30.05.2007
(73) Patentinhaber: Biotest AG, D-63303 Dreieich (DE)
(72) Erfinder: KRETSCHMAR, Michael, 63500 Seligenstadt (DE); MÖLLER, Wolfgang, 61440 Oberursel (DE)
(74) Vertreter: Kalhammer, Georg
(86) Internationale Anmeldenummer: PCT/EP2005/009730
(87) Internationale Veröffentlichungsnummer: WO 2006/029775

(56) Entgegenhaltungen:
- US-A- 4 774 323
- US-B1- 6 465 624
- DATABASE BIOSIS [Online] BIOSCIENCES INFORMATION SERVICE, PHILADELPHIA, PA, US; 1983, ZYKOVA T A ET AL: "A SIMPLE AND EFFECTIVE ADDITIONAL STEP IN PURIFICATION OF BOVINE BLOOD SERUM FIBRONECTIN" XP002352960 Database accession no. PREV198478048777 & VOPROSY MEDITSINSKOI KHIMII, Bd. 25, Nr. 5, 1983, Seiten 114-117, ISSN: 0042-8809

## Beschreibung

Die Erfindung betrifft ein Verfahren zur Hetstellung einer Zusammensetzung enthaltend einen Gerinnungsfaktor, umfassend die Abreicherung von Fibronektin aus Plasmafraktionen bei niedriger Ionenstärke durch Verschiebung des pH auf einen niedrigen Wert, so dass Fibronektin präzipitiert und der Lösung entzogen wird.

Fibronektin besteht aus zwei Polypeptidketten (α und β-Kette) mit einem Molekulargewicht von jeweils ca. 220 kDa. Seine lösliche Form kommt im Plasma und in anderen Körperflüssigkeiten vor, während seine unlösliche Form in der extrazellulären Matrix und in der Unterschicht von Membranen zu finden ist. Die Konzentrationen des Fibronektin im Humanplasma beträgt durchschnittlich 270 µg/ml. Aufgrund seiner Affinität zu Zelloberflächen und vielen verschiedenen Makromolekülen, besitzt es eine Vielzahl von Funktionen, z.B. Zell-Zell-Adhäsion, Zell-Substrat-Adhäsion, Zellteilung, Wundheilung u.v.a.. Die hohe Affinität zu Oberflächen und die Fähigkeit vernetzte Strukturen aufzubauen führt zu Problemen bei der Fraktionierung von Humanplasma zu therapeutischen Produkten. Es können Plasmafraktionen entstehen, deren hoher Gehalt an Fibronektin Filtrationen, Ultrafiltrationen und Chromatographie-Schritte zum Herstellen eines hochreinen Plasmaproduktes, z.B. eines Konzentrates an Gerinnungsproteinen deutlich erschwert. Häufig äußert sich das Problem im frühzeitigen Absättigen bzw. Blockieren von Filtern und Chromatographie-Säulen. Die als Gegenmaßnahme angewandte Vergrößerung der Filtrationsfläche oder Chromatographie-Säulenvolumina ist mit einer deutlichen Erhöhung der Herstellkosten verbunden.

Verschiedene Verfahren zur Herstellung isolierter Fibronektin-Fraktionen wurden beschrieben:

Horowitz et al. (Transfusion 24, 357-362 (1984): Preparation of antihemophilic factor and fibronectin from human plasma cryoprecipitate) beschreiben ein Verfahren, bei dem ein Kryopräzipitatextrakt als Ausgangsmaterial dient. Nach einer Aluminiumhydroxid-Fällung wird der lösliche Überstand idealerweise bei 10°C und einem pH von 6,5 inkubiert, wobei Fibronektin ausfällt und über Zentrifugation entfernt wird. Um eine hochreine Fibronektin-Präparation herzustellen, wird das wieder gelöste Fibronektin an eine Gelatine-Sepharose-Säule gebunden und mittels Natriumbromid enthaltenden Puffer bei pH 5,5 eluiert.

Ingham et al. (Molecular Immunology 20, 287-295 (1983): Interaction of plasma fibronectin with gelatin and complement C1q) stellen eine Untersuchung vor, bei der gezeigt wurde, dass Fibronektin in Anwesenheit von Gelatine aufgrund von spezifischen Interaktionen selektiv mit Polyethylenglykol (PEG) 4000 aus Lösungen gefällt werden kann. Dabei ergab sich, dass bei einer Gelatine-Konzentration von 0,4 mg/ml schon eine PEG 4000-Konzentration von 3% ausreicht, um 50% des Fibronektin zu fällen, während in Abwesenheit von Gelatine dazu 11 % benötigt werden.

Das Europäische Patent 0011231 B1 stellt ein Verfahren vor, um kälteunlösliches Globulin (Fibronektin) durch Zugabe von 1,8 - 2,6 mol/l Aminosäuren, vorzugsweise Glycin, und 8 - 12% (w/v) Neutralsalz bei Temperaturen >18°C zu fällen und vom Überstand zu trennen.

US-Patent 4,406,886 beschreibt eine Methode zur Herstellung eines Faktor VIII-Präparates, bei der durch Zugabe von Zinksalzen Fibrinogen und Fibronektin in Form von Zink-Komplexen gefällt und entfernt werden.

Das US-Patent 4,278,594 stellt ein Verfahren zur Herstellung einer gereinigten Fibronektin-Fraktion vor. Dabei wird in einem Plasmapool unter Zuhilfenahme von Heparin Fibrinogen und Fibronektin gefällt. Das Präzipitat wird gelöst, auf eine DEAE-Zellulose-Säule aufgetragen und Fibrinogen und Fibronektin selektiv eluiert.

Im Europäischen Patent EP 0503991 B1 werden für die Herstellung eines von Willebrand Faktor-Präparates zwei Anionenaustauscher-Chromatographie-Schritte mit einer Gelatine-Affinitätschromatographie kombiniert. Dabei dient die Affinitätssäule vor allem der Abtrennung von Fibronektin.

Im US-Patent 5,981,254 wird ein Verfahren zur Herstellung eines Thrombin-Produktes beschrieben, das eine Fällung von Fibrinogen, Fibronektin und Faktor XIII beinhaltet. Dabei wird durch Zusatz von hohen Salzkonzentrationen bei neutralen pH-Werten eine Fällung erzielt. Alternativ wird beschrieben, die Fällung durch den Zusatz von sauren Salzen zu erzielen, die zu einem erniedrigten pH führen. Dieses Verfahren führt zu relativ hohen Ionenstärken, die für weitere Prozessschritte reduziert werden müssen.

Die beschriebenen Verfahren sind relativ aufwendig, insbesondere wenn ausschließlich eine Abtrennung des Fibronektins von der Wertfraktion als Ziel gesetzt wird. Häufig werden kostenintensive Affinitätschromatographien durchgeführt. Einfacher ist eine Fällung, die allerdings meist durch Zusetzen von z.B. Polyethylenglykol oder hohen Salzkonzentrationen erzielt wird. Der Zusatz von Fällreagenzien und hohen Salzkonzentrationen erhöhen die Gefahr, dass das gewünschte Produkt (z.B. von Willebrand Faktor) zum Teil mit aus dem Überstand gefällt wird.

Eine Aufgabe der vorliegenden Erfindung ist die Bereitstellung eines einfachen Verfahrens zur Abtrennung von Fibronektin aus Plasmafraktionen, so dass eine weitere Prozessierung zur Aufreinigung des Zielproteins erleichtert wird. Überraschenderweise hat sich gezeigt, dass durch eine einfache Titration auf einen niedrigen pH-Wert Fibronektin gefällt werden kann. Besonders überraschend war, dass die gebildeten Fibronektin-Fäden sich beim Rühren um den Rührer wickelten, dort einen "Klot" bildeten und so sehr einfach aus der Lösung entfernt werden konnten.

Die vorliegende Erfindung betrifft daher ein Verfahren zur Herstellung einer Zusammensetzung enthaltend einen Gerinnungsfaktor, dadurch gekennzeichnet, dass man
(i) den pH-Wert einer Plasmafraktion auf unter pH 5,4 einstellt, so dass sich ein Niederschlag bildet, wobei die Ionenstärke unter 500 mM liegt, und
(ii) den gebildeten Niederschlag abtrennt.

Der Ausdruck "Plasmafraktion" bezeichnet eine Zusammensetzung, die aus Plasma erhalten wurde und verschiedene Plasmaproteine enthält. Die Plasmafraktion, die als Ausgangszusammensetzung in Schritt (i) eingesetzt wird, ist eine flüssige Zusammensetzung. Vorzugsweise ist die flüssige Zusammensetzung eine Lösung oder eine Suspension, am bevorzugtesten ist die Zusammensetzung eine Lösung.

In einer besonderen Ausführungsform ist die Plasmafraktion gelöstes Kryopräzipitat. Dieses gelöste Kryopräzipitat kann durch verschiedene Verfahren vorgereinigt sein. Beispiele sind Aluminiumhydroxid-Behandlung, Solvens-/Detergens-Behandlung und/oder Anionenaustauschchromatographie.

Die Konzentration an Natriumchlorid oder Kaliumchlorid in der Plasmafraktion ist vorzugsweise 50 bis 250 mM, bevorzugter 100 bis 200 mM, am bevorzugtesten 120 bis 150 mM.

Die Ionenstärke der Plasmafraktion liegt nach Schritt (i) und vor Schritt (ii) vorzugsweise unter 500 mM, bevorzugter unter 300 mM, am bevorzugtesten unter 200 mM. Vorzugsweise wird durch die Einstellung des pH-Werts in Schritt (i) die Ionenstärke nicht stark erhöht, d.h. sie bleibt vorzugsweise niedriger als 500 mM, bevorzugter niedriger als 300 mM, am bevorzugtesten niedriger als 200 mM.

Die Plasmafraktion kann beispielsweise folgende Puffersubstanzen enthalten: Citrationen, Acetationen, Phosphationen und/oder Aminosäuren.

Aminosäurezusätze werden vorzugsweise in Konzentrationen eingesetzt, die nicht zu einer Proteinfällung ohne pH-Verschiebung führen. Dies bedeutet beispielsweise für Glycin, dass die Konzentration unter 1,8 M liegt, vorzugsweise unter 500 mM, bevorzugter unter 200 mM, am bevorzugtesten unter 150 mM.

Die Konzentration an Fibronektin in der Plasmafraktion, die Schritt (i) unterworfen wird, ist in der Regel mindestens 0,05 g/l, vorzugsweise wenigstens 0,1 g/l, noch bevorzugter wenigstens 0,25 g/l, am bevorzugtesten wenigstens 0,5 g/l. Die Konzentration an Fibronektin in der Plasmafraktion kann beispielsweise 0,1 bis 5 g/l, vorzugsweise 0,1 bis 2 g/l sein.

Erfindungsgemäß wird in dem beanspruchten Verfahren der pH-Wert der Plasmafraktion auf unter pH 5,4 eingestellt. Dabei bildet sich ein Niederschlag, der Fibronektin enthält. Vorzugsweise wird der pH-Wert auf unter pH 5,3 eingestellt, noch bevorzugter auf unter pH 5,2. Der eingestellte pH-Wert liegt somit vorzugsweise in einem Bereich von pH 4,5 bis unter 5,4, bevorzugt in einem Bereich von pH 4,7 bis 5,3, bevorzugter in einem Bereich von pH 4,8 bis 5,2, noch bevorzugter in einem Bereich von pH 4,9 bis 5,1.

In der Regel wird das Einstellen des pH-Werts durch Zugabe einer sauren Komponente erreicht. Als saure Komponente können verschiedene Säuren eingesetzt werden, beispielsweise Salzsäure, Phosphorsäure oder Essigsäure.

Die saure Komponente wird üblicherweise über einen bestimmten Zeitraum zugegeben, beispielsweise tropfenweise. Somit wird nach und nach ein pH-Wert im oben näher definierten Bereich eingestellt ("titriert").

Während und nach dem Einstellen des pH-Werts wird die Plasmafraktion vorzugsweise in Bewegung gehalten oder durchmischt, beispielsweise durch Rühren. Es ist weiterhin bevorzugt, dass nach Einstellen des pH-Werts die Plasmafraktion für einen bestimmten Zeitraum weiter durchmischt wird (z.B. durch Rühren), im allgemeinen für wenigstens 10 Minuten, vorzugsweise für wenigstens 20 Minuten, am bevorzugtesten für einen Zeitraum von 30 bis 90 Minuten. In diesem Zeitraum bilden sich klebrige Aggregate, die zu einem erheblichen Anteil Fibronektin erhalten. Daher ist es gemäß einer bevorzugten Ausführungsform vorgesehen, dass ein geeigneter Rührer, z.B. ein Anker- oder Paddelrührer eingesetzt wird, an dessen Rührblatt sich der Niederschlag anheftet. Das präzipitierte Fibronektin kann somit leicht der Lösung entzogen werden.

Das erfindungsgemäße Verfahren kann in einem breiten Temperaturspektrum durchgeführt werden, z.B. von ca. 1 °C bis ca. 37°C. Bevorzugte Temperaturbereiche sind 4 bis 35°C, bevorzugter 10 bis 30°C, am bevorzugtesten wird das Verfahren bei 20 bis 25°C durchgeführt.

Die für das erfindungsgemäße Verfahren eingesetzte Plasmafraktion ist vorzugsweise ein gelöstes Kryopräzipitat, das durch dem Fachmann an sich bekannte Verfahren hergestellt worden ist. So kann das gelöste Kryopräzipitat durch einen oder mehrere der Schritte Aluminiumhydroxid-Behandlung, Solvens-/Detergens-Behandlung und Anionenaustauschchromatographie vorgereinigt sein. Die Durchführung dieser Verfahren ist dem Fachmann an sich bekannt.

Durch das erfindungsgemäße Verfahren zur Entfernung von Fibronektin aus Plasmafraktionen kann die Konzentration an Fibronektin in der Plasmafraktion um wenigstens 50% verringert werden. Vorzugsweise wird die Konzentration an Fibronektin in der Plasmafraktion um 70 bis 99%, bevorzugter um 80 bis 99%, am bevorzugtesten um 90 bis 98% oder um 95 bis 98% verringert.

In einer besonderen Ausführungsform liegt der Verlust an Zielprotein, beispielsweise an VWF, bei höchstens 50%, bevorzugt bei höchstens 40%, bevorzugter bei höchstens 30%, noch bevorzugter bei höchstens 20%, am bevorzugtesten bei höchstens 10%.

Nach Abtrennung des Fibronektin enthaltenden Niederschlags aus der Plasmafraktion können weitere Reinigungsschritte erfolgen, um wenigstens einen Gerinnungsfaktor aufzureinigen. Vorzugsweise wird der Gerinnungsfaktor von Willebrand Faktor (VWF) weiter aufgereinigt. In einer besonderen Ausführungsform wird nach der pH-Füllung zur Abtrennung von Fibronektin eine Hydroxylapatit-Chromatographie durchgeführt. Hydroxylapatit ist eine Form von Calciumphosphat mit der Zusammensetzung Ca₅(PO₄)₃OH bzw. Ca₁₀(PO₄)₆OH₂, die als stationäre Phase für die Chromatographie von Proteinen, Nukleinsäuren und anderen Makromolekülen eingesetzt werden kann. Neben der kristallinen Form von Hydroxylapatit kann auch eine keramische Form verwendet werden, welche durch Sintern erhältlich ist. Hydroxylapatit kann beispielsweise von der Firma Bio-Rad (München, Deutschland) bezogen werden. Deren keramisches Hydroxylapatit wird in zwei Formen (Typ 1 und Typ 2) zur Verfügung gestellt. Typ 1-Material hat aufgrund größerer Oberflächen eine höhere Bindungskapazität für kleinere Moleküle, z.B. kleine Proteine. Dagegen weist das Typ 2-Material größere Poren in den Partikeln auf, die ein Eindringen und damit eine bessere Bindung von großen Molekülen, z.B. DNA oder großen Proteinen, ermöglicht. Die Materialien weisen vorzugsweise folgende Eigenschaften auf:

**Tabelle 1**

| | Dynamische Bindungskapazität | Nominale Porendurchmesser |
|---|---|---|
| Typ 1 | >13,7 mg Lysozym/ml CHT* | 600-800 Å |
| Typ 2 | >6,8 mg Lysozym/ml CHT* | 800-1000 Å |

| | | |
|---|---|---|
| *CHT=Ceramic Hydroxy Apatite | | |

Kristallines oder keramisches Hydroxylapatit ist frei verfügbar. Verfahren zu ihrer Herstellung sind im Stand der Technik bekannt.

In einer ersten Variante umfasst die Hydroxylapatit-Chromatographie, dass (i) die Plasmafraktion nach Abtrennung des gefällten Fibronektin-Niederschlags mit einer Hydroxylapatit-Matrix in Kontakt gebracht wird, so dass Fibrinogen und/oder Fibronektin an die Hydroxylapatit-Matrix gebunden wird, während VWF im wesentlichen nicht an die Hydroxylapatit-Matrix gebunden wird, und gegebenenfalls anschließend (ii) ungebundener von Willebrand Faktor (VWF) von der Hydroxylapatit-Matrix getrennt wird. Diese Variante wird in der vorliegenden Anmeldung als "Durchlaufchromatographie" bezeichnet, da VWF nicht an die Hydroxylapatit-Matrix bindet. Das Verfahren kann in Form einer Säulenchromatographie oder im Batch-Verfahren durchgeführt werden; die Durchführung einer Säulenchromatographie ist bevorzugt. Im Fall einer Säulenchromatographie befindet sich VWF im Durchlauf und wenigstens ein verunreinigendes Protein, z. B. Fibronektin und/oder Fibrinogen, wird an das Hydroxylapatit gebunden.

Die Hydroxylapatit-Chromatographie wird gemäß dieser ersten Variante bei einem pH-Wert von 6,5 bis 8,5, vorzugsweise von 6,8 bis 8,5, bevorzugter von 6,8 bis 7,5, am bevorzugtesten von 7,0 bis 7,5 durchgeführt. Üblicherweise haben Lauf-, Wasch- und Elutionspuffer, sowie die aufzutragende Proteinlösung den gleichen pH-Wert. Es sind aber auch Varianten praktikabel, bei denen diese Lösungen unterschiedlichen pH-Werte aufweisen. Die Zusammensetzung, die mit der Hydroxylapatitmatrix in Kontakt gebracht wird, enthält vorzugsweise Natriumphosphat und/oder Kaliumphosphat. Die Gesamtkonzentration an Natriumphosphat und/oder Kaliumphosphat in der Lösung ist beispielsweise 0 bis 100 mM, vorzugsweise 10 bis 50 mM, am bevorzugtesten 20 bis 40 mM, d. h. als Laufpuffer kann eine Pufferlösung mit den genannten Konzentrationen eingesetzt werden. Die Zusammensetzung wird bei einer niedrigen Salzkonzentration von 0 - 100 mM Kalium- oder Natriumphosphat, bevorzugt bei 10 - 50 mM, bei einem pH von vorzugsweise 6,8 bis 8,5, besonders bevorzugt bei einem pH von 7,0 - 7,5, auf eine Hydroxylapatit-Säule aufgetragen. Das Hydroxylapatit ist in dieser Variante vorzugsweise keramisches Hydroxylapatit, besonders bevorzugt vom Typ 1, wie von Bio-Rad (München, Deutschland) vertrieben. Unter diesen Bedingungen bindet der Großteil der VWF-Moleküle nicht an die Matrix und befindet sich im Durchlauf, während Verunreinigungsproteine, wie z.B. Fibrinogen oder Fibronektin, zum Großteil an die Matrix binden.

Durch die Durchlaufchromatographie können Präparationen erhalten werden, die nur geringe Mengen an Fibrinogen und Fibronektin enthalten. In der Regel ist die Konzentration an Fibrinogen-Antigen in der Durchlauffraktion niedriger als 25 µg/ml, bevorzugt niedriger als 15 µg/ml, bevorzugter niedriger als 10 µg/ml, am bevorzugtesten höchstens 5 µg/ml. Die Konzentration an Fibronektin-Antigen in der Durchlauffraktion ist üblicherweise niedriger als 250 µg/ml, bevorzugt niedriger als 150 µg/ml, bevorzugter niedriger als 100 µg/ml, am bevorzugtesten höchstens 50 µg/ml. Die Konzentration an Fibrinogen-Antigen und Fibronektin-Antigen kann durch an sich bekannte Verfahren bestimmt werden, z. B. wie in den Beispielen der vorliegenden Anmeldung beschrieben.

Die Konzentration an Fibrinogen in der Durchlauffraktion ist vorzugsweise niedriger als 10%, bevorzugter niedriger als 5%, noch bevorzugter niedriger als 2,5% der Konzentration an Fibrinogen in der Auftragslösung (vor Durchlaufchromatographie). Die Konzentration an Fibronektin in der Durchlauffraktion ist vorzugsweise niedriger als 10%, bevorzugter niedriger als 5%, noch bevorzugter niedriger als 2,5% der Konzentration an Fibronektin in der Auftragslösung (vor Durchlaufchromatographie). Die Durchlaufchromatographie ist besonders zur Aufreinigung von VWF geeignet. So ist die VWF-Ausbeute der Durchlaufchromatographie (bezogen auf die Massenbilanz) in der Regel höher als 50%, bevorzugt höher als 60%, am bevorzugtesten höher als 75%. Die spezifische Aktivität (Ristocetin-Cofaktor-Aktivität pro mg Gesamtprotein) kann durch die Durchlaufchromatographie um wenigstens 100%, vorzugsweise um wenigstens 150%, am bevorzugtesten um wenigstens 200% erhöht werden.

Bei einer Aufreinigung von VWF ist eine zweite Variante der Hydroxylapatit-Chromatographie besonders vorteilhaft. In dieser zweiten Variante wird VWF an die Hydroxylapatit-Matrix gebunden und anschließend eluiert. Diese Variante wird in der vorliegenden Anmeldung als "bindende Chromatographie" bezeichnet. Üblicherweise umfasst die bindende Chromatographie, dass
(a) VWF an die Hydroxylapatit-Matrix gebunden wird,
(b) Verunreinigungen bei niedrigerer Salzkonzentration herausgewaschen werden und
(c) anschließend die VWF-haltige Wertfraktion bei höherer Salzkonzentration eluiert wird.

In Schritt (a) wird eine Lösung, die VWF und ein oder mehrere verunreinigende Proteine enthält, mit der Hydroxylapatit-Matrix in Kontakt gebracht. Die Gesamtkonzentration an Natrium- und/oder Kaliumphosphat in dieser Lösung ist üblicherweise 0 bis 200 mM, vorzugsweise 1 bis 200 mM, bevorzugter 1 bis 50 mM, am bevorzugtesten 10 bis 30 mM.

In dem Waschschritt (b) wird die Hydroxylapatit-Matrix mit einem Puffer niedriger Salzkonzentration gewaschen. Die Gesamtkonzentration an Natrium- und/oder Kaliumphosphat in diesem Waschpuffer ist in der Regel 100 bis 300 mM, vorzugsweise 150 bis 250 mM, am bevorzugtesten 180 bis 240 mM.

In Schritt (c) kann die VWF-haltige Wertfraktion mit einem Puffer höherer Salzkonzentration eluiert werden. Der Elutionspuffer enthält in der Regel 200 bis 500 mM, vorzugsweise 250 bis 400 mM Natrium- und/oder Kaliumphosphat.

Durch Verschiebung der Salzkonzentrationen können Ausbeute und Reinheit verändert werden. Je höher die Salzkonzentration im Waschpuffer ist, desto sauberer ist die erhaltene Wertfraktion. Die VWF-Ausbeute erniedrigt sich dadurch allerdings. Weiterhin beeinflusst der gewählte pH-Wert die optimale Salzkonzentration für den Waschpuffer. Je niedriger der pH-Wert ist, desto stärker ist die Bindung von VWF an die Hydroxylapatit-Matrix. Entsprechend können die gewählten Salzkonzentrationen bei niedrigen pH-Werten höher sein, bei höheren pH-Werten hingegen niedriger. Die (bindende) Hydroxylapatit-Chromatographie wird bei einem pH-Wert von 5 bis 7,5, vorzugsweise von 5,5 bis unter 6,8, am bevorzugtesten von 6,0 bis 6,5 durchgeführt. Üblicherweise haben Lauf-, Wasch- und Elutionspuffer, sowie die aufzutragende Proteinlösung den gleichen pH-Wert. Es sind aber auch Varianten praktikabel, bei denen diese Lösungen unterschiedlichen pH-Werte aufweisen.

In dieser zweiten Variante wird die VWF-haltige Lösung, z.B. die Plasmafraktion nach pH-Fällung und Abtrennung des Fibronektin-Niederschlags, bei einer niedrigen Salzkonzentration, vorzugsweise 0 - 100 mM Kalium- oder Natriumphosphat, besonders bevorzugt bei 10 - 30 mM, bei einem pH-Wert von 5,5 - 6,8, bevorzugt 6,0 - 6,5, auf eine Hydroxylapatit-Säule, z.B. keramisches Hydroxylapatit Typ 2 aufgetragen. Der Großteil der VWF-Moleküle wird unter diesen Bedingungen gebunden. Durch Waschen mit einer Lösungen mit höherer Salzkonzentration mit z.B. Kalium- oder Natriumphosphat, beispielsweise 230 mM Natriumphosphat pH 6,0, können Verunreinigungen, z.B. Fibronektin, ausgewaschen werden. Die Wertfraktion wird anschließend mit hochkonzentrierten Salzlösungen, z.B. Phosphatlösungen, wie z.B. 400 mM Natriumphosphat pH 6,0, eluiert.

Durch die bindende Chromatographie können VWF-Präparationen erhalten werden, die praktisch keine nachweisbaren Mengen an Fibrinogen und Fibronektin mehr enthalten. Wenn die Auftragslösung, die mit der Hydroxylapatit-Matrix in Kontakt gebracht wird, eine Plasmafraktion ist und/oder Fibrinogen oder Fibronektin enthält, kann eine praktisch quantitative Entfernung der verunreinigenden Proteine Fibrinogen und Fibronektin aus der Lösung erzielt werden. So ist die Konzentration an Fibrinogen in der Elutionsfraktion vorzugsweise niedriger als 25% der Konzentration an Fibrinogen in der Auftragslösung (vor der bindenden Chromatographie). Die Konzentration an Fibronektin in der Elutionsfraktion ist vorzugsweise niedriger als 10%, bevorzugter niedriger als 5% der Konzentration an Fibronektin in der Auftragslösung (vor der bindenden Chromatographie). Die Konzentrationen an Fibrinogen bzw. Fibronektin in der Elutionsfraktion (Wertfraktion) sind in der Regel unterhalb der Nachweisgrenze von ca. 1 µg/ml.

Durch die bindende Hydroxylapatit-Chromatographie können VWF-Präparationen mit hoher spezifischer Aktivität erhalten werden. Die spezifische Aktivität in der Elutionsfraktion kann höher als 50 E/mg Protein sein, bevorzugt ist sie höher als 75 E/mg Protein, bevorzugter höher als 85 E/mg Protein, am bevorzugtesten wenigstens 100 E/mg Protein. Die VWF-Aktivität wird bestimmt mit dem Ristocetin-Cofaktor-Assay, der die Bindungsfähigkeit von VWF an den Plättchenrezeptor Glycoprotein Ib/IX unter dem Einfluss des Antibiotikums Ristocetin ermittelt. Die spezifische VWF-Aktivität kann bestimmt werden wie in den Beispielen beschrieben.

Für die Herstellung eines besonders reinen VWF-Präparates können die beiden beschriebenen Varianten der Hydroxylapatit-Chromatographie miteinander oder mit anderen Reinigungstechniken kombiniert werden. Als besonders geeignet hat sich nämlich gezeigt, zunächst nach dem oben beschriebenen Verfahren eine Durchlaufchromatographie mit Hydroxylapatit zur Abreicherung der Hauptverunreinigungen durchzuführen. Anschließend wird die Wertfraktion beispielsweise mit 1 M HCl auf pH 6,0 titriert. Die Probe wird, wie für die bindende Chromatographie beschrieben, auf eine Hydroxylapatit-Säule aufgetragen. VWF-Moleküle werden gebunden und selektiv eluiert. In einer dritten Variante der Hydroxylapatit-Chromatographie wird daher zunächst eine Durchlaufchromatographie mit Hydroxylapatit durchgeführt, wobei VWF nicht an die Hydroxylapatit-Matrix bindet, und anschließend die Durchlauffraktion unter bindenden Bedingungen rechromatographiert und die VWF-Fraktion eluiert. Für die Durchlaufchromatographie ist es sinnvoll, aber nicht notwendig, Phosphationen als Puffersubstanz zu verwenden. Für die Elution von VWF bei der bindenden Chromatographie ist Phosphat ein spezifisches Agens.

Die in dieser Anmeldung beschriebenen Ausführungsformen können miteinander kombiniert werden.

Lösungen, die mit dem hier beschriebenen Verfahren von Fibronektin befreit werden können, sind Plasmafraktionen, aus denen andere Komponenten aufgereinigt werden sollen. Dies können Plasmafraktionen sein, aus denen beispielsweise Gerinnungspräparate, wie der von Willebrand Faktor, gewonnen wird. Die Plasmafraktionen, die mit diesem Verfahren aufgearbeitet werden können, enthalten als Hauptverunreinigung Fibronektin, das in Konzentrationen von > 0,1 g/l vorliegt. Die Lösung wird durch Zugabe einer sauren Komponente, beispielsweise 1 M Salzsäure auf einen pH-Wert < 5,4, vorzugsweise 5,4 bis 4,8, besonders bevorzugt 5,2 bis 5,0, titriert. Unter Rühren wird die Lösung > 10 Minuten, bevorzugt 30 bis 90 Minuten inkubiert. Es bilden sich klebrige Aggregate, die sich bei Verwendung eines geeigneten Rührers, z.B. eines Anker- oder eines Paddelrührers, an das Rührblatt heften und so der Lösung entzogen werden. Dies hat den Vorteil, dass ein Großteil des Niederschlags nicht durch technisch aufwendige und kostenintensive Weise mittels Filtration oder Zentrifugation entfernt werden muss. Die Effektivität der Fadenbildung wird durch die Ionenstärke beeinflusst. Sehr gute Ergebnisse wurden beispielsweise mit Pufferlösungen erzielt, die 100 - 200 mM NaCl, bevorzugt 120 - 150 mM NaCl enthielten. Das Verfahren kann in einem breiten Temperatur-Spektrum von, z.B. 4°C bis 35°C durchgeführt werden. Bevorzugt wird das Verfahren bei Raumtemperatur durchgeführt. Die Abreicherungseffektivität für Fibronektin hängt von der Zusammensetzung der Lösung ab und liegt typischerweise zwischen 70% und 95%.

Die nachfolgenden Ausführungsbeispiele erläutern die Erfindung näher.

### Beispiel 1: Fällen von Fibronektin im Labormaßstab bei verschiedenen Temperaturen

Als Ausgangsmaterial wurde eine Kryopräzipitat-Lösung verwendet, die durch Aluminiumhydroxid-Fällung, Polysorbat 80 / TNBP-Behandlung und AnionenaustauschChromatographie, wie z.B. im WO 9315105 A1 beschrieben, vorgereinigt wurde. Für die Isolierung des enthaltenen von Willebrand Faktors (VWF) musste zunächst störendes Fibronektin entfernt werden. Die Lösung enthielt als Hauptkomponenten 0,18 g/l VWF-Antigen (VWF-Ag), 0,05 g/l Fibrinogen-Antigen und 1,49 g/l Fibronektin-Antigen. Die Lösung enthielt folgende Puffersubstanzen: 10 mM Citrat, 160 mM NaCl 120 mM Glycin, 1 mM CaCl₂. Jeweils 1 l der Lösung wurden bei 4°C, 20°C bzw. 35°C unter Rühren durch Zugabe von 1 M HCl auf pH 5,2 titriert. Während des Inkubierens für 60 min wickelten sich in allen 3 Fällen weiße Fibronektin-Fäden um den Rührer und bildeten einen massiven Klot. Die verbleibende Lösung ließ sich problemlos mit einem Membranfilter klarfiltrieren.

**Tabelle 2: Zusammensetzung der Ausgangsprobe und der gefällten 4°C-, 20°C- und 35°C-Probe**

| | VWF-Ag [g/l] | Fibrinogen-Ag [g/l] | Fibronektin-Ag [g/l] |
|---|---|---|---|
| Ausgang | 0,18 | 0,05 | 1,49 |
| 4°C | 0,14 | 0,01 | 0,09 |
| 20°C | 0,15 | 0,02 | 0,04 |
| 35°C | 0,16 | 0,02 | 0,1 |

Es zeigte sich, dass im Temperaturbereich von 4°C bis 35°C eine pH-Shift-Präzipitation hervorragend geeignet ist, um Fibronektin in großen Mengen abzutrennen. Der Verlust am Zielprotein VWF liegt hier bei maximal 22% (4°C) bei einer Abreicherung an Fibronektin von mindestens 93% bis 97%.

Die Konzentration an VWF-Antigen wurde mit Hilfe des STA^{®} Compact der Firma Diagnostica Stago (Roche Diagnostics, Mannheim) und deren Test-Reagenzien (STA LIA vWF) bestimmt.

Für die Bestimmung der Menge an Fibrinogen-Antigen und Fibronektin-Antigen wurden nephelometrische Methoden zur quantitativen Bestimmung der Fibrinogen-Antigen- und Fibrinogen-Antigen-Konzentration im Beckman-Array^{®} 360 (Beckman Coulter, Monheim) verwendet.

### Beispiel 2: Abtrennung von Fibronektin bei einem präparativen Ansatz

Es wurde eine Proteinlösung verwendet, die wie in Beispiel 1 vorgereinigt wurde und im selben Puffersystem vorlag. 40 I dieser Lösung wurden bei Raumtemperatur auf pH 5,2 titriert und für 60 min unter Rühren inkubiert. Fibronektin-Aggregate wurden größtenteils durch Anheften an das Rührblatt des Rührers aus der Lösung entfernt. Nach Klarfiltration über einen Membranfilter konnte die Lösung zur Gewinnung eines VWF-Präparates weiterverarbeitet werden.

**Tabelle 3: Fällung von Fibronektin in einem präparativen 40 I-Ansatz**

| | VWF-Ag [g/l] | Fibrinogen-Ag [g/l] | Fibronektin-Ag [g/l] |
|---|---|---|---|
| Ausgang | 0,20 | 0,07 | 1,33 |
| nach Titration und Filtration | 0,19 | 0,03 | 0,13 |

Bei einem Verlust von nur 5% an dem Zielprotein VWF konnten 90% des Fibronektins entfernt werden.

### Ergänzende Literaturstellen

Folgende Literaturstellen zu verschiedenen Analytikmethoden werden ergänzend genannt:

### VWF-Aktivität:

Veyradier A, Fressinaud E, Meyer D (1998): Laboratory diagnosis of von Willebrand disease. Int J Lab Res 28 (4): 201-210.

### VWF-Antigen:

Budde U, et al. (1984): Acquired von Willebrand's disease in the myeloproliferative syndrome. Blood 64 (5): 981-985.

Newman DJ, Henneberry H, Price CP (1992): Particle enhanced light scattering immunoassay. Ann Clin Biochem 29 (Pt1): 22-42.

### Fibronektin-Antigen:

Sandberg L, et al. (1985): Plasma fibronectin levels in acute and recovering malnourished children. Clin Physiol Biochem. 3(5):257-264.

Colli A, et al. (1986): Diagnostic accuracy of fibronectin in the differential diagnosis of ascites. Cancer.: 58(11):2489-2493.

### Fibrinogen-Antigen:

Ernst E, Resch KL (1993): Fibrinogen as a cardiovascular risk factor: a meta-analysis and review of the literature. Ann Intern Med.: 118(12):956-963.

Jelic-Ivanovic Z, Pevcevic N (1990): Fibrinogen determination by five methods in patients receiving streptokinase therapy. Clin Chem.: 36(4):698-699.

## Patentansprüche

1. Verfahren zur Herstellung einer Zusammensetzung enthaltend einen Gerinnungsfaktor, umfassend dass man
(i) den pH-Wert einer Plasmafraktion auf unter pH 5,4 einstellt, so dass sich ein Niederschlag bildet, wobei die Ionenstärke unter 500 mM liegt und
(ii) den gebildeten Niederschlag abtrennt.

2. Verfahren nach Anspruch 1, **dadurch gekennzeichnet, dass** der pH-Wert der Plasmafraktion auf einen Wert zwischen pH 4,7 und pH 5,3 einstellt wird.

3. Verfahren nach Anspruch 1 oder 2, **dadurch gekennzeichnet, dass** die Ionenstärke unter 300 mM liegt.

4. Verfahren nach einem der Ansprüche 1 bis 3, **dadurch gekennzeichnet, dass** die Ionenstärke unter 200 mM liegt.

5. Verfahren nach einem der Ansprüche 1 bis 4, **dadurch gekennzeichnet, dass** die Plasmafraktion nach Einstellen des pH-Werts in Schritt (i) für mindestens 10 Minuten gerührt wird.

6. Verfahren nach einem der Ansprüche 1 bis 5, **dadurch gekennzeichnet, dass** die Abtrennung des Großteils des Fibronektin-Niederschlags mit dem Rührblatt eines Rührers erfolgt.

7. Verfahren nach einem der Ansprüche 1 bis 6, **dadurch gekennzeichnet, dass** die Fibronektinkonzentration in der Plasmafraktion vor Schritt (i) mindestens 0,1 g pro Liter ist.

8. Verfahren nach einem der Ansprüche 1 bis 7, **dadurch gekennzeichnet, dass** die Konzentration von NaCl oder KCI in der Plasmafraktion 100 - 200 mM ist.

9. Verfahren nach einem der Ansprüche 1 bis 8, **dadurch gekennzeichnet, dass** die Ausgangslösung Glycin in einer Konzentration unter 500 mM enthält.

10. Verfahren nach einem der Ansprüche 1 bis 9, **dadurch gekennzeichnet, dass** die Ausgangslösung Glycin in einer Konzentration unter 200 mM enthält.

11. Verfahren nach einem der Ansprüche 1 bis 10, **dadurch gekennzeichnet, dass** die Ausgangslösung Glycin in einer Konzentration von 50 bis 200 mM enthält.

12. Verfahren nach einem der Ansprüche 1 bis 11, **dadurch gekennzeichnet, dass** die Ausgangslösung Glycin in einer Konzentration von 100 bis 150 mM enthält.

13. Verfahren nach einem der Ansprüche 1 bis 12, **dadurch gekennzeichnet, dass** die Plasmafraktion gelöstes Kryopräzipitat ist.

14. Verfahren nach Anspruch 13, **dadurch gekennzeichnet, dass** das gelöste Kryopräzipitat durch Aluminiumhydroxid-Behandlung, Solvent-/Detergent-Behandlung und Anionenaustauschchromatographie vorgereinigt ist.

15. Verfahren nach einem der Ansprüche 1 bis 14, **dadurch gekennzeichnet, dass** man nach Schritt (ii) wenigstens einen Gerinnungsfaktor aufreinigt.

16. Verfahren nach Anspruch 15, **dadurch gekennzeichnet, dass** der Gerinnungsfaktor von Willebrand Faktor ist.

## Claims

1. A process for the production of a composition containing a coagulation factor, comprising
(i) adjusting the pH of a plasma fraction to below pH 5.4 so as to form a precipitate, the ionic strength being below 500 mM, and
(ii) separating the precipitate formed.

2. The process according to claim 1, **characterized in that** the pH of the plasma fraction is adjusted to a value between pH 4.7 and pH 5.3.

3. The process according to any of claims 1 or 2, **characterized in that** the ionic strength is below 300 mM.

4. The process according to any of claims 1 to 3, **characterized in that** the ionic strength is below 200 mM.

5. The process according to any of claims 1 to 4, **characterized in that** after adjusting the pH value in step (i) the plasma fraction is stirred for at least 10 minutes.

6. The process according to any of claims 1 to 5, **characterized in that** the majority of the fibronectin precipitate is separated by means of the agitator blade of a stirrer.

7. The process according to any of claims 1 to 6, **characterized in that** before step (i) the fibronectin concentration in the plasma fraction is at least 0.1 g per liter.

8. The process according to any of claims 1 to 7, **characterized in that** the concentration of NaCl or KCI in the plasma fraction is 100 - 200 mM.

9. The process according to any of claims 1 to 8, **characterized in that** the starting solution contains glycine at a concentration below 500 mM.

10. The process according to any of claims 1 to 9, **characterized in that** the starting solution contains glycine at a concentration below 200 mM.

11. The process according to any of claims 1 to 10, **characterized in that** the starting solution contains glycine at a concentration of 50 to 200 mM.

12. The process according to any of claims 1 to 11, **characterized in that** the starting solution contains glycine at a concentration of 100 to 150 mM.

13. The process according to any of claims 1 to 12, **characterized in that** the plasma fraction is dissolved cryoprecipitate.

14. The process according to claim 13, **characterized in that** the dissolved cryoprecipitate is previously purified by aluminum hydroxide treatment, solvent/detergent treatment and anion exchange chromatography.

15. The process according to any of claims 1 to 14, **characterized in that** after step (ii) at least one coagulation factor is purified.

16. The process according to claim 15, **characterized in that** the coagulation factor is von Willebrand factor.

## Revendications

1. Procédé de préparation d'une composition contenant un facteur de coagulation, comprenant les étapes consistant à :
(i) ajuster le pH d'une fraction de plasma à moins de 5,4, de sorte qu'un précipité se forme, la force ionique étant inférieure à 500 mM et
(ii) séparer le précipité ainsi obtenu.

2. Procédé selon la revendication 1, **caractérisé en ce que** le pH de la fraction de plasma est ajusté à une valeur comprise entre 4,7 et 5,3.

3. Procédé selon la revendication 1 ou 2, **caractérisé en ce que** la force ionique est inférieure à 300 mM.

4. Procédé selon l'une quelconque des revendications 1 à 3, **caractérisé en ce que** la force ionique est inférieure à 200 mM.

5. Procédé selon l'une quelconque des revendications 1 à 4, **caractérisé en ce que** la fraction de plasma est agitée pendant au moins 10 minutes après ajustement du pH dans l'étape (i).

6. Procédé selon l'une quelconque des revendications 1 à 5, **caractérisé en ce que** la séparation de la majeure partie du précipité de fibronectine est mise en oeuvre avec la pale d'agitation d'un agitateur.

7. Procédé selon l'une quelconque des revendications 1 à 6, **caractérisé en ce que** la concentration en fibronectine dans la fraction de plasma avant l'étape (i) est d'au moins 0,1 g par litre.

8. Procédé selon l'une quelconque des revendications 1 à 7, **caractérisé en ce que** la concentration du NaCl ou du KCl dans la fraction de plasma est de 100 à 200 mM.

9. Procédé selon l'une quelconque des revendications 1 à 8, **caractérisé en ce que** la solution de départ contient de la glycine en une concentration inférieure à 500 mM.

10. Procédé selon l'une quelconque des revendications 1 à 9, **caractérisé en ce que** la solution de départ contient de la glycine en une concentration inférieure à 200 mM.

11. Procédé selon l'une quelconque des revendications 1 à 10, **caractérisé en ce que** la solution de départ contient de la glycine en une concentration de 50 à 200 mM.

12. Procédé selon l'une quelconque des revendications 1 à 11, **caractérisé en ce que** la solution de départ contient de la glycine en une concentration de 100 à 150 mM.

13. Procédé selon l'une quelconque des revendications 1 à 12, **caractérisé en ce que** la fraction de plasma est un cryoprécipité dissous.

14. Procédé selon la revendication 13, **caractérisé en ce que** le cryoprécipité dissous est épuré au préalable par un traitement à l'hydroxyde d'aluminium, un traitement au solvant/détergent et une chromatographie échangeuse d'anions.

15. Procédé selon l'une quelconque des revendications 1 à 14, **caractérisé en ce que** qu'au moins un facteur de coagulation est purifié après l'étape (ii).

16. Procédé selon la revendication 15, **caractérisé en ce que** le facteur de coagulation est du facteur von Willebrand.
